(19) **Europäisches Patentamt**

**European Patent Office**

**Office européen des brevets**

(11) Publication number: **0 037 666**
**B2**

(12) # NEW EUROPEAN PATENT SPECIFICATION

(45) Date of publication of the new patent specification: **09.12.87**

(51) Int. Cl.⁴: **C 08 L 67/00,** C 08 K 5/09, C 07 C 53/126, C 07 C 51/347

(21) Application number: **81301209.3**

(22) Date of filing: **20.03.81**

(54) Thermoplastic polyester resin composition.

(30) Priority: **01.04.80 JP 42250/80**

(43) Date of publication of application:
**14.10.81 Bulletin 81/41**

(45) Publication of the grant of the patent:
**13.06.84 Bulletin 84/24**

(45) Mention of the opposition decision:
**09.12.87 Bulletin 87/50**

(84) Designated Contracting States:
**CH DE FR GB LI NL**

(56) References cited:
**FR - A - 2 129 162**
**US - A - 4 029 682**

**CHEMICAL ABSTRACTS, vol. 91, no. 12, 17th September 1979, page 37, abstract 92482p Columbus, Ohio, US**

**The file contains technical information submitted after the application was filed and not included in this specification**

(73) Proprietor: **MITSUBISHI CHEMICAL INDUSTRIES LIMITED, 5-2, Marunouchi 2-chome Chiyoda-ku, Tokyo 100 (JP)**

(72) Inventor: **Hayashi, Masahiro, 1-4 Narusedai 3-chome Machida-shi, Tokyo (JP)**
Inventor: **Yoshimura, Tamotsu, 19-9, Tadao 3-chome Machida-shi, Tokyo (JP)**
Inventor: **Mukai, Seiichi, 861-2,Shimoasoh Tama-ku, Kawasaki-shi Kanagawa-ken (JP)**
Inventor: **Shikama, Masaharu, 5-6 Tsutsujigaoka Midori-ku, Yokohama-shi Kanagawa-ken (JP)**
Inventor: **Kusumoto, Hideto, 12-3 Ogawa 1-chome Machida-shi, Tokyo (JP)**
Inventor: **Yamanouchi, Hideki, 32-7, Fukazawa 4-chome Setagaya-ku, Tokyo (JP)**

(74) Representative: **Warden, John Christopher et al, R.G.C. Jenkins & Co. 12-15, Fetter Lane, London EC4A 1PL (GB)**

## Description

The present invention relates to a thermoplastic polyester resin composition comprising polyethylene terephthalate. More particularly, it relates to a thermoplastic polyester resin composition comprising polyethylene terephthalate which rapidly crystallizes and which comprises a specific crystallization accelerator.

Thermoplastic polyesters, especially polyethylene terephthalate are important as raw materials for preparing fibers, films and molded products. They have excellent characteristics such as high wearing resistance, excellent creep property and high dimensional accuracy because of their partial crystalline structure. Therefore, they are especially suitable for molded products to which severe mechanical stress is applied or which are heated under severe conditions.

Polyethylene terephthalate, however has a slow crystallization velocity and therefore requires a relatively high mold temperature (about 140°C) and relatively long pressing time in the preparation of a molded product. This is disadvantageous in the preparation of a molded product by an injection molding process.

If the crystallization velocity can be increased, the molding cycle can be shortened to reduce the overall cost of production. This is a remarkable advantage.

Chemical abstracts Vol. 91 No. 12, p.37, 92482p discloses thermoplastic polyester resin compositions having improved mold release which contain C>27 alkanoic acids, their esters or salts, e.g. poly(tetramethyleneterephthalate) and 0.1 ethylene glycol montanate.

FR-A-2 129 162 discloses polyethylene terephthalate molding compositions including a dispersed nucleating agent such as a sodium stearate and potassium stearate.

According to the present invention there is provided a thermoplastic polyester resin composition comprising polyethylene terephthalate which composition is quickly crystallizable and which comprises 0.1 to 10 wt. parts, per 100 wt. parts of thermoplastic polyester, of a sodium salt of an α-alkyl fatty acid having at least 26 carbon atoms obtained by an addition of a fatty acid having at least 3 carbon atoms to an α-olefin having at least 23 carbon atoms.

Various crystallization accelerators for crystallizing thermoplastic polyesters at high crystallization velocity have been studied, and it has been found that sodium salts of certain α-alkyl higher fatty acids are remarkably effective as crystallization accelerators.

The thermoplastic polyesters which may be used as raw materials in the present invention comprise polyethylene terephthalate and derivatives thereof wherein the terephthalic acid component or glycol component is partially substituted by another comonomer component. Suitable comonomer components include at least one bifunctional carboxylic acid such as isophthalic acid, naphthalenedicarboxylic acid, 4,4'-diphenoxyethanedicarboxylic acid, adipic acid or sebacic acid; and at least one glycol such as ethyleneglycol, trimethyleneglycol, tetramethyleneglycol, hexamethyleneglycol, and decamethyleneglycol. Preferred polyethylene terephthalates include polyethylene terephthalates having at least 80 mol% of ethylene terephthalate repeat units.

The thermoplastic polyesters usually have an intrinsic viscosity of 0.5 to 2.0 (in a mixed solvent of phenol and tetrachloroethane at 1:1 by weight; at 30°C).

The sodium salts of α-alkyl fatty acids having at least 26 and preferably 30-400 carbon atoms which are used as crystallization accelerators for thermoplastic polyesters in the present invention (hereinafter referred to as higher fatty acid sodium salts) are produced by bonding an olefin having one terminal double bond (hereinafter referring to as an α-olefin) to a fatty acid, suitable by a conventional process (for example as disclosed in Yukagaku Vol. 19, page 121, 1970) and heating the product in an aqueous solution of sodium hydroxide to form the sodium salt.

The α-olefin must have at least 23 carbon atoms. When a mixture of α-olefins having different numbers of carbon atoms is used, the average number of carbon atoms must be at least 23. It is preferable to use a mixture of α-olefins having an average of at least 30 carbon atoms such as Dialene 30® commercially available from Mitsubishi Chemical Ind. (average of 43 carbon atoms measured by iodine value) and Dialene 208® commercially available from Mitsubishi Chemical Ind. (average of 23 carbon atoms). The fatty acid is preferably propionic acid. The higher fatty acids obtained by bonding these components can be a mixture of α-methyl fatty acids having at least 33 carbon atoms (typically an average of 46 carbon atoms measured by acid value; (referred to as α-Fatty acid 30) and a mixture of α-methyl fatty acids having carbon atoms of 23-31 (such as an average of 26 carbon atoms measured by acid value; (referred to as α-Fatty acid 208)). The fatty acid is not limited to propionic acid and can be another fatty acid such as butyric acid, valeric acid.

In accordance with the present invention, 0.1 to 10 wt. parts of the higher fatty acid sodium salt is incorporated into 100 wt. parts of the thermoplastic polyester. When the content of the higher fatty acid sodium salt is less than 0.1 wt. part, the effect of the invention is not satisfactorily attained whereas when it is more than 10 wt. parts, the mechanical property of the polyethylene terephthalate as the matrix is inferior. These are disadvantageous. When a higher fatty acid sodium salt having less than 26 carbon atoms is incorporated, the side effect for thermal degradation of the thermoplastic polyester is disadvantageous even though the same content as Na content of the higher fatty acid salt is incorporated in the thermoplastic polyester as shown in Reference.

In the preparation of the polyester resin composition, the crystallization accelerator is blended with the polyethylene terephthalate and they are melted and mixed. The melt-mixing can be the conventional process using an extruder or the other desired machine.

In the composition of the present invention, it is possible to add a desired additive which is usually added, such as inorganic fillers, glass fibers and plasticizers. The additive can be added at any desired stage by a conventional manner.

The present invention will be further illustrated by the following examples.

In the examples and references, acid value was measured by Standard Oil and Fat Analysis Test method published by Nippon Yukagaku Kyokai (1978).

Preparation 1

Preparation of α-Fatty acid 30:

Into a reactor, 167.5 g. of Dialene® 30 (α-olefin having an average of 43 carbon atoms manufactured and sold by Mitsubishi Chemical Ind.) and 185 g. of propionic acid were charged and the mixture was heated at 135 to 140°C in nitrogen atmosphere and then 9.2 g. of di-t-butyl peroxide was added dropwise during 4 hours. The mixture was stirred at the same temperature for 10 hours. The unreacted propionic acid was distilled off under a reduced pressure to obtain 178.6 g. of a mixture of higher fatty acids (average carbon atoms 46) comprising α-methylmonocarboxylic acids having at least 33 carbon atoms as main components. The product is referred to as α-Fatty acid 30. The α-Fatty acid 30 had an acid value of 38.7 KOH mg./g. and a melting point of 70 to 72°C.

Preparation 2

Preparation of α-Fatty acid 208:

In accordance with the process of Preparation 1, except using Dialene® 208 (α-olefin having an average of 23 carbon atoms manufactured and sold by Mitsubishi Chemical Ind.) instead of Dialene® 30, a higher fatty acid having an average of 26 carbon atoms, an acid value of 102.4 KOH mg./g. and a melting point of 52–54°C was produced. The product is referred to as α-Fatty acid 208.

Preparation 3

Preparation of α-Fatty acid 168:

In accordance with the process of Preparation 1 except for using Dialene® 168 (α-olefin having an average of 17 carbon atoms (16–18 carbon atoms manufactured and sold by Mitsubishi Chemical Ind.) instead of Dialene® 30, a higher fatty acid having average of 20 carbon atoms, an acid value of 179 KOH mg./g. and a melting point of 48–50°C was produced. The product is referred to as α-Fatty acid 168.

Preparation 4

Preparation of Higher Fatty Acid Salts:

A mixture of 100 g. of α-Fatty acid 30 obtained in Preparation 1 and 10 ml. of xylene was heated to 140°C and then, 9 g. of 30% aqueous solution of sodium hydroxide was added dropwise during about 1 hour with removal of water out of the system by heating with stirring. After removing about 7.5 ml. of water, xylene was distilled off under a reduced pressure to obtain 115 g. of sodium salt of α-Fatty acid 30 (hereinafter referring to as α-Fatty acid salt 30).

In accordance with the above-mentioned process except for using α-Fatty acid 208 or α-Fatty acid 168 instead of α-Fatty acid 30, the process was carried out to obtain sodium salt of α-Fatty acid 208 or sodium salt of α-Fatty acid 168 (hereinafter referred to as α-Fatty acid salt 208 and α-Fatty acid salt 168).

Examples 1 to 6 and references 1 to 7

Polyethylene terephthalate ($[\eta]$ = 0.66) (NOVA-PET manufactured and sold by Mitsubishi Chemical Ind.; (hereinafter referred to as PET) and the higher fatty acid salts obtained in Preparation 4 and sodium stearate or sodium montanate were mechanically blended at ratios shown in Table 1 and each mixture was melted and mixed by a uniaxial extruder equipped with a Dulmage screw having a diameter of 20 mm (L/D=28) and the resulting strand was cooled in water and cut to form pellets. The pellet was tested by using a differential calorimeter (referred to as DSC; Perkin-Elmer IB type) by heating and cooling it to measure a crystallization temperature (Tcc) on heating and a melting temperature (Tm) and a crystallization temperature ($Tc^{300}$) on cooling after melting at 300°C for 5 minutes.

When the crystallization velocity is higher, $Tc^{300}$ is higher and heat absorption peak on a DSC test paper for $Tc^{300}$ is sharp. In order to show the sharpness, a triangle along the heat absorption peak is drawn and an index of the ratio heat/base of the triangle (the base of the triangle is the base line of the peak) is given.

When Tcc is higher, melt fluidity of a polymer at a low temperature is higher and a molded product obtained by molding it in a low temperature mold has higher crystallinity.

When a sodium salt of a relatively lower fatty acid is used, thermal degradation of PET in a matrix is highly accelerated. The fact is studied in view of a stability of a resulting strand (stable melt spinning) and an intrinsic viscosity $[\eta]$ which is measured in a mixed solvent of phenol/tetrachloroethane (1:1 by weight) at 30°C. The results of the examples and references are shown in Table 1.

Sodium montanate as one of higher fatty acid imparts excellent crystallization acceleration effect, but it causes remarkable deterioration of thermal stability of PET as the matrix.

## Table 1

| Example or Reference | Ref. 1 | Ref. 2 | Ref. 3 | Ref. 4 | Ref. 5 |
|---|---|---|---|---|---|
| Kind of crystallization accelerator | none | Na-stearate | Na-stearate | α-Fatty acid salt 168 | Na-montanate |
| Amount of the accelerator (wt.%) | 0 | 1 | 3 | 3 | 4 |
| Stability of strand * | ○ | ○ | × | △ | — |
| $[\eta]$ of strand | 0.602 | 0.497 | | 0.378 | 0.48 |
| Result by DSC: | | | | | |
| Tcc (°C) | 142 | 117 | | | 114 |
| Tm (°C) | 259 | 260 | | 258 | 255 |
| $Tc^{300}$ (°C) | 187 | 217 | | 219 | 215 |
| Ratio of height/base of peak | 1.3 | 5.1 | | 3.1 | — |

## Table 1 (continued)

| Example or Reference | Ref. 6 | Ref. 7 | Exp. 1 | Exp. 2 | Exp. 3 |
|---|---|---|---|---|---|
| Kind of crystallization accelerator | Na-montanate | Na-montanate | α-Fatty acid salt 208 | α-Fatty acid salt 208 | α-Fatty acid salt 208 |
| Amount of the accelerator (wt.%) | 3 | 1 | 1 | 3 | 5 |
| Stability of strand * | — | — | ○ | ○ | ○ |
| $[\eta]$ of strand | 0.49 | 0.53 | 0.559 | 0.569 | 0.542 |
| Result by DSC: | | | | | |
| Tcc (°C) | 116 | 117 | 120 | 119 | 118 |
| Tm (°C) | 258 | 258 | 257 | 260 | 260 |
| $Tc^{300}$ (°C) | 213 | 211 | 211 | 218 | 220 |
| Ratio of height/base of peak | — | — | 4.5 | 6.3 | 7.8 |

## Table 1 (continued)

| Example or Reference | Exp. 4 | Exp. 5 | Exp. 6 |
|---|---|---|---|
| Kind of crystallization accelerator | α-Fatty acid salt 30 | α-Fatty acid salt 30 | α-Fatty acid salt 30 |
| Amount of the accelerator (wt.%) | 1 | 3 | 5 |
| Stability of strand * | ○ | ○ | ○ |
| $[\eta]$ of strand | 0.562 | 0.615 | 0.548 |
| Result by DSC: | | | |
| Tcc (°C) | 121 | 123 | 121 |
| Tm (°C) | 257 | 257 | 260 |
| $Tc^{300}$ (°C) | 213 | 213 | 213 |
| Ratio of height/base of peak | 7.4 | 5.3 | 7.4 |

Note: * Stability of strand:
    ○: A strand can be extruded in stable condition.
    △: A strand is extruded in pulsation.
    ×: A strand is intermittently extruded.

**Claims:**

1. A thermoplastic polyester resin composition comprising polyethylene terephthalate which composition is quickly crystallizable and which comprises 0.1 to 10 wt. parts, per 100 wt. parts of thermoplastic polyester, of a sodium salt of an α-alkyl fatty acid having at least 26 carbon atoms obtained by an addition of a fatty acid having at least 3 carbon atoms to an α-olefin having at least 23 carbon atoms.

2. A thermoplastic polyester resin composition according to claim 1 wherein said α-olefin has at least 30 carbon atoms.

3. A thermoplastic polyester resin composition according to claim 2 wherein said sodium fatty acid salt has 30 to 400 carbon atoms.

4. A thermoplastic polyester resin composition according to any preceding claim wherein said fatty acid is propionic acid, butyric acid or valeric acid.

5. A thermoplastic polyester resin composition according to any preceding claim wherein said resin composition comprises polyethylene terephthalate having at least 80 mol% ethyleneterephthalate repeating units.

**Patentansprüche**

1. Thermoplastische Polyesterharzmasse, welche rasch kristallisierbar ist und pro 100 Gew.-Teile des thermoplastischen Polyesters 0,1 bis 10 Gew.-Teile eines Natriumsalzes einer α-Alkylfettsäure mit mindestens 26 Kohlenstoffatomen, erhalten durch eine Addition einer Fettsäure mit mindestens 3 Kohlenstoffatomen an ein α-Olefin mit mindestens 23 Kohlenstoffatomen, umfasst.

2. Thermoplastische Harzmasse nach Anspruch 1, wobei das α-Olefin mindestens 30 Kohlenstoffatome aufweist.

3. Thermoplastische Polyesterharzmasse nach Anspruch 2, wobei das Natriumfettsäuresalz 30 bis 400 Kohlenstoffatome aufweist.

4. Thermoplastische Harzmasse nach irgendeinem der vorstehenden Ansprüche, wobei die Fettsäure Propionsäure, Buttersäure oder Valeransäure ist.

5. Thermoplastische Polyesterharzmasse nach irgendeinem der vorstehenden Ansprüche, wobei die Harzmasse Polyäthylenterephthalat mit mindestens 80 Mol-% Äthylenterephthalatstruktureinheiten umfasst.

**Revendications**

1. Composition de résine de polyester thermoplastique, comprenant du polyéthylènetéréphthalate et qui est rapidement cristallisable et comprend 0,1 à 10 parties en poids, pour 100 parties en poids de polyester thermoplastique d'un sel de sodium d'un acide gras α-alkylique contenant au moins 26 atomes de carbone obtenu par addition d'un acide gras contenant au moins 3 atomes de carbone à une α-oléfine contenant au moins 23 atomes de carbone.

2. Composition de résine de polyester thermoplastique selon la revendication 1, dans laquelle ladite α-oléfine contient au moins 30 atomes de carbone.

3. Composition de résine de polyester thermoplastique selon la revendication 2, dans laquelle ledit sel de sodium d'acide gras contient 30 à 400 atomes de carbone.

4. Composition de résine de polyester thermoplastique selon l'une quelconque des revendications précédentes, dans laquelle ledit acide gras est l'acide propionique, l'acide butyrique ou l'acide valérique.

5. Composition de résine de polyester thermoplastique selon l'une quelconque des revendications précédentes, comprenant du polyéthylènetéréphtalate contenant au moins 80% en mole de motifs récurrents d'éthylènetéréphthalate.